# EUROPÄISCHE PATENTSCHRIFT

(11) **EP 1 374 928 B1**
(45) Veröffentlichungstag und Bekanntmachung des Hinweises auf die Patenterteilung: **05.12.2007**
(21) Anmeldenummer: 03012887.0
(22) Anmeldetag: 06.06.2003
(51) Int. Cl.: A61M 1/10, F04D 3/00

(54) **Blutpumpe mit einem Impeller**
Blood pump comprising an impeller
Pompe à sang à turbine

(30) Priorität: 21.06.2002 DE 10227886; 12.07.2002 DE 10231479
(43) Veröffentlichungstag der Anmeldung: 02.01.2004
(73) Patentinhaber: Mückter, Helmut, 52076 Aachen (DE)
(72) Erfinder: Mückter, Helmut, 52076 Aachen (DE)
(74) Vertreter: Castell, Klaus

(56) Entgegenhaltungen:
- EP-A- 0 560 000
- WO-A-97/49439
- WO-A-99/34847
- US-A- 4 995 857
- US-A- 5 267 940
- US-B1- 6 247 892

## Beschreibung

Die Erfindung betrifft eine Blutpumpe mit einem Impeller. Zur Unterstützung des Herzens oder auch als Ersatz eines Herzens werden verschiedenartigste Pumpen eingesetzt. Diese Pumpen dienen als mechanisches Implantat zur teilweisen Unterstützung oder zum vollständigen Ersatz der Funktion einer oder beider Herzkammern oder zur Förderung des Blutstromes in einem Blutgefäß.

Als Pumpe werden einerseits Membranpumpen mit integrierten Klappenventilen eingesetzt, die mechanisch, hydraulisch oder pneumatisch betrieben werden. Andererseits werden Impellerpumpen verschiedenster Ausführungen mit radialen, halbaxialen bzw. diagonalen oder axialen Laufrädern verwendet. Derartige Pumpen sind in der WO 97/49439 und in der US 4,994,078 beschrieben. Diese Pumpen werden mit unterschiedlichen Kreislaufanschlusstechniken implantiert. Dabei handelt es sich vorwiegend um folgende Grundprinzipien:
1. Vollständiger Ersatz des Herzens durch ein Pumpensystem. Hierbei wird das geschädigte Herz entfernt und durch ein mechanisches Kunstherz ersetzt.
2. Bypasssysteme. Hierbei wird das geschädigte Herz in situ belassen und die Blutströmung über Kanülen ganz oder teilweise an einer oder beiden Herzkammern vorbei durch ein intra- oder extrakorporales Pumpsystem geführt.
3. Intravasale Impellersysteme. Hierbei wird eine kleine Impellerpumpe, welche in eine Kanüle integriert ist, über die Hauptschlagader retrograd durch die Aortenklappe hindurch in die linke Herzkammer geführt. Der Blutstrom wird durch die Impellerpumpe aus der Herzkammer in die Hauptschlagader geleitet.

Die Art der Ausbildung der Pumpen und deren Einsatz in der Medizintechnik führen jedoch zu unterschiedlichen Problemen.

Thrombembolische Komplikationen: Durch die großen Fremdoberflächen der meisten verfügbaren Systeme und insbesondere der unabdingbar notwendigen Kanülen kommt es auch unter höchstmöglicher medikamentöser Hemmung der Blutgerinnung zur Ausbildung von Blutgerinnseln.

Blutungskomplikationen: Die bei den meisten Systemen erforderliche medikamentöse Hemmung der Blutgerinnung in hoher Dosierung zur Vermeidung thrombembolischer Komplikationen kann ihrerseits wiederum zu schwerwiegenden, lebensbedrohlichen Blutungen führen.

Unzureichende Sicherheit bei Systemausfall: Bei dem aus dem Stand der Technik bekannten Pumpensystemen ist bereits ein sehr kurzzeitiger Ausfall der Pumpe akut lebensbedrohlich.

Entzündungsrisiko: Durch große Oberflächen der verwendeten Pumpen besteht die Gefahr der Ansiedlung von Bakterien auf den Kunststoffoberflächen.

Beeinträchtigung der Herzfunktion durch das Pumpensystem: Die meisten bekannten Pumpsysteme führen bauartbedingt zu einer ungünstigen Beeinflussung der Herzfunktion. Zum einen ist dies auf großvolumige Kanülen zurückzuführen, die das Blut aus dem Vorhof oder der Ventrikelspitze entnehmen, und dort den Blutfluss oder die Funktion der Herzklappen beeinträchtigen. Zum anderem sind dies Kanülen, welche durch die Herzklappen hindurch geschoben werden, und dort die Herzklappenfunktion selbst beeinträchtigen und den freien Strömungsquerschnitt der Herzklappen behindern.

Aus der US 5,267,940 ist eine Blutpumpe mit einem Impeller bekannt, wobei zwei direkt am Pumpengehäuse angeordnete Anschlüsse an zwei Blutgefäße außerhalb des Herzens angeschlossen werden. Die Anschlüsse sind Verbindungseinrichtungen in Form von Stutzen. Die Druckschrift beschreibt eine Anordnung der Blutpumpe im Bereich der Aorta descendens mit zwei schlauchartigen Anschlussstücken, welche es ermöglichen, die Pumpe neben der eigentlichen Implantationsstelle einzusetzen.

Die WO 97/49439 A1 zeigt eine Blutpumpe mit einem langgestreckten rohrförmigen Pumpengehäuse, in welchem ein Pumpenrad von einem Motor angetrieben wird, wobei das Pumpengehäuse an einem Ende einen Einlass und am entgegengesetzten Ende einen Auslas aufweist. Der Einlass und der Auslass sind ebenfalls als Stutzen geformte Verbindungseinrichtungen.

Die US 4,995,857 beschreibt eine Blutpumpe mit einem Impeller, wobei aus einem Pumpengehäuse zwei Leitungen herausragen, die geeignet sind, mit jeweils einem peripheren Blutgefäß verbunden zu werden. Diese Leitungen sind Schlauchstutzen.

Die EP 0 560 000 A2 offenbart eine Blutpumpe mit einem Impeller und zwei Verbindungseinrichtungen in Form von Schlauchstutzen. Eine dieser Verbindungseinrichtungen in Form von Schlauchstutzen soll an die Aorta angeschlossen werden, während die zweite dazu vorgesehen ist, an das Herz angeschlossen zu werden. Dabei sind die beiden Schlauchstutzen baugleich ausgeführt.

Die US 6,247,892 B1 schlägt eine Axialpumpe vor, welche ein Fluid kontinuierlich fördert, wobei zwei Motoren und zwei gegensinnig bewegte Impeller vorgesehen sind, um jegliche seitlich wirkenden Kräfte der Pumpe zu eliminieren.

Der Erfindung liegt die Aufgabe zu Grunde, eine Blutpumpe mit einem Impeller derart weiterzubilden, dass sie unmittelbar in einen Gefäßabschnitt einsetzbar ist, und zwar ohne die Verwendung von Anschlusskanülen.

Diese Aufgabe löst eine Blutpumpe mit einem Impeller mit mindestens zwei Verbindungseinrichtungen in Form eines Nahtrings oder einer Gefäßprothese zur Interposition in ein Blutgefäß außerhalb des Herzens im Bereich der Aorta ascendens oder des truncus pulmonalis oder zwischen dem truncus pulmonalis und den Pulmonalarterien.

Diese Gefäßverbindungseinrichtungen ermöglichen es, die Impellerpumpe direkt mit dem Gefäß zu verbinden, ohne dass Kanülen verwendet werden müssen. Dies ermöglicht den Einsatz der Blutpumpe zwischen zwei Gefäßenden wie beispielsweise nach einer Sektion eines Aortenabschnittes oder im Bereich einer Gefäßgabelung.

Das Pumpensystem ist in der Lage, die Funktion einer oder beider Herzkammern vollständig oder teilweise zu ersetzen. Außerdem wird eine wirksame Druckentlastung in einer oder beiden Herzkammern erzielt, wodurch die Rückbildung einer Dilatation der Herzhöhle oder der Herzhöhlen wirksam unterstützt wird. Die Möglichkeit der Anordnung der Pumpe außerhalb des Herzens führt dazu, dass die Blutströmung durch die Herzhöhlen während der Pumpunterstützung aufrechterhalten werden kann und eine intracavitäre Stase vermieden wird. Dadurch wird einer Gerinnselbildung in den Herzhöhlen entgegengewirkt. Die Ausbildung der Pumpe ermöglicht sogar den Einsatz des Pumpsystems bei implantierten mechanischen Herzklappenprothesen in Aorten- oder Pulmonalposition. Das direkte Anbringen von Gefäßverbindungseinrichtungen an der Blutpumpe und der Verzicht auf Kanülen führt zu einer sehr geringen Baugröße der Pumpe und zu minimalen Fremdoberflächen. Dadurch wird das Risiko einer Gerinnselbildung und einer bakteriellen Besiedlung an den Fremdoberflächen so gering wie möglich gehalten. Durch die Reduzierung der mit dem Blut oder Gewebe in Verbindung stehenden Oberfläche werden auch die Kosten für die benötigten hoch biokompatiblen Materialien minimal gehalten.

Darüber hinaus kann die vorgeschlagene Pumpe so implantiert werden, dass sie bei ausreichender Erholung der Herzfünktion auf einfache Art und Weise operationstechnisch entfernt werden kann.

Die Pumpe ist so ausgebildet, dass sie zur Unterstützung der linken Herzkammer in Form eines Pumpenconduits in die Aorta ascendens, zur Unterstützung der rechten Herzkammer in Form eines Pumpenconduits in den Truncus pulmonalis oder in die Pulmonalisgabel und zur Förderung des Blutstromes oder zur Druckerhöhung in einem bestimmten Gefäßabschnitt in das zuführende Gefäß implantiert werden kann. Bei peripherer arterieller Verschlusskrankheit, bei der Dialyse oder zur extrakorporalen Membranoxygenation (ECMO) wird die Blutpumpe in Form eines Pumpenconduits in das diesen Gefäßabschnitt versorgende Hauptgefäß implantiert. Bei der Entlastung einer oder beider Herzkammern ist die Pumpe vorzugsweise so zu implantieren, dass die Funktion der natürlichen Herzklappen (Aorten- bzw. Pulmonalklappe) oder die Funktion einer gegebenenfalls an deren Stelle implantierten Herzklappenprothese durch das implantierte Pumpenconduit nicht beeinträchtigt wird.

Vorteilhaft ist es, wenn die Gefäßverbindungseinrichtungen direkt am Pumpengehäuse angeordnet sind. Diese Anordnung der Gefäßverbindungseinrichtungen direkt am Pumpengehäuse führt zu einer Blutpumpe mit einem Pumpengehäuse und einem darin angeordneten elektromechanisch angetriebenen Impeller, wobei die Blutpumpe mindestens zwei Verbindungseinrichtungen zum Anschluss an ein Blutgefäß außerhalb des Herzens, wie beispielsweise einen Nahtring oder eine Gefäßprothese aufweist, welche derart gestaltet sind, dass ein Anschluss des Blutgefäßes unmittelbar am Pumpengehäuse ermöglicht wird. Hierdurch wird ein kompakter Aufbau der Pumpe und eine Reduktion der benötigten Teile erzielt.

Ein besonders kompakter Aufbau wird dadurch erzielt, dass die Länge des Pumpengehäuses kürzer als zweimal vorzugsweise 1,5-mal dessen Durchmesser ist. Dies führt dazu, dass bei der Implantation der Blutpumpe nur ein kurzes Aderstück entfernt werden muss, das durch die Blutpumpe im Sinne eines Conduits ersetzt wird.

Vorteilhaft ist es auch, wenn die Länge der Gefäßverbindungseinrichtung besonders kurz ausgebildet ist, um nur einen kurzen Aderbereich bei der Implantation beschädigen oder entfernen zu müssen. Die Gefäßverbindungseinrichtung sollte vorzugsweise kürzer als der Durchmesser des Zwischenstücks sein.

Ein einfacher Aufbau der Pumpe wird dadurch erzielt, dass sie einen Motor und ein Pumpengehäuse mit vorzugsweise als Leitschaufeln ausgebildeten Stegen aufweist, wobei die vorzugsweise als Leitschaufeln ausgebildeten Stege zwischen dem Motor und dem äußeren Mantel des Pumpengehäuses angeordnet sind. Die Leitschaufeln können somit den Motor vorzugsweise koaxial zum Pumpengehäuse halten und gleichzeitig eine Leitfunktion auf den durchgeleiteten Blutstrom ausüben.

Die Leitschaufeln sind vorzugsweise derart stabil ausgebildet, dass sie auch bei auftretenden Dralländerungen den Motor sicher im Pumpengehäuse halten.

Zur Stromversorgung des Motors wird vorgeschlagen, dass hierzu erforderliche Kabel oder Metallstifte innerhalb der vorzugsweise als Leitschaufeln ausgelegten Stege verlaufen. Dadurch kommen die Stromzuführungsleitungen mit dem Blut nicht in Berührung.

Vorteilhaft ist es, wenn die Blutpumpe so gestaltet ist, dass bei einem Systemausfall neben der fehlenden Herzunterstützung keine weiteren systembedingten negativen Einflüsse auf den Kreislauf und die Lungenfunktion zu erwarten sind. Um dies zu erreichen wird vorgeschlagen, dass der freie Strömungsquerschnitt zwischen dem Motor und dem äußeren Mantel des Pumpengehäuses mindestens 50 %, vorzugsweise mindestens 80 % des freien Strömungsquerschnitts an einem Ende des Zwischenstücks beträgt. Auch bei einem Ausfall der Pumpe kann somit das Blut nahezu ungehindert durch die im Sinne eines Conduits ausgebildete Blutpumpe hindurch fließen, wodurch eine Kreislauffunktion aufrechterhalten bleiben kann.

Ein besonderes Ausführungsbeispiel der erfindungsgemäßen Pumpe sieht vor, dass sie zwei Motoren, zwei Pumpengehäuse und eine Gehäuseverbindung aufweist. Eine derartige vorzugsweise adaptierbare Verbindungseinrichtung kann bei optimaler Auslegung der Pumpen dazu führen, dass nur geringfügige äußere Drehmomente entstehen.

Hierzu wird vorgeschlagen, dass die Impeller gegenläufig antreibbar sind. Dies kann beispielsweise durch gegenläufig angesteuerte Motoren erreicht werden. Hierbei werden die Massenträgheitsmomente der rotierenden Teile vorzugsweise so ausgelegt, dass bei dynamischem bzw. pulsatilem Betrieb möglichst kleine äußere Drehmomente resultieren.

Ein anderes Ausführungsbeispiel sieht vor, dass die Blutpumpe ein Pumpengehäuse mit einem Zusatzmotor, welcher eine Masse gegenläufig zum Impeller rotierend antreibt, aufweist. Auch hierdurch können die äußeren Momente gering gehalten werden. Ein derartiges Ausführungsbeispiel sieht vor, dass ein weiterer elektromechanischer Antrieb konnektiert ist, welcher eine rotierende Masse gegenläufig und synchron zur Drehrichtung der Rotorwelle der Blutpumpe antreibt, und dessen Massenträgheitsmoment so ausgelegt ist, dass bei einer Dralländerung keine oder nur geringfügige äußere Momente resultieren.

Vorteilhaft ist es, wenn die Blutpumpe ein Pumpengehäuse mit einer Aufhängeeinrichtung aufweist, die es ermöglicht, die Blutpumpe in einem Gewebe, vorzugsweise am knöchernem Brustkorb zu befestigen. Hierzu wird vorgeschlagen, dass an das Pumpengehäuse eine vorzugsweise adaptierbare Aufhängeeinrichtung konnektiert wird, welche die Erstellung einer Verbindung zu umliegenden Gewebestrukturen erlaubt, die in der Lage sind, die bei dynamischem oder pulsatilem Betrieb auftretenden Momente aufzunehmen.

Drei verschiedene Ausführungsbeispiele einer Blutpumpe sind in der Zeichnung dargestellt und werden im folgenden näher erläutert.

Es zeigt
- Figur 1: einen Schnitt durch eine in die Aorta ascendens implantierte Blutpumpe,
- Figur 2: eine teilweise geschnittene Ansicht einer Blutpumpe mit zwei Pumpengehäusen zur Entlastung beider Herzkammern und
- Figur 3: eine teilweise geschnittene Ansicht einer Blutpumpe zur Implantation in die Gabelung des Pulmonalisstammes.

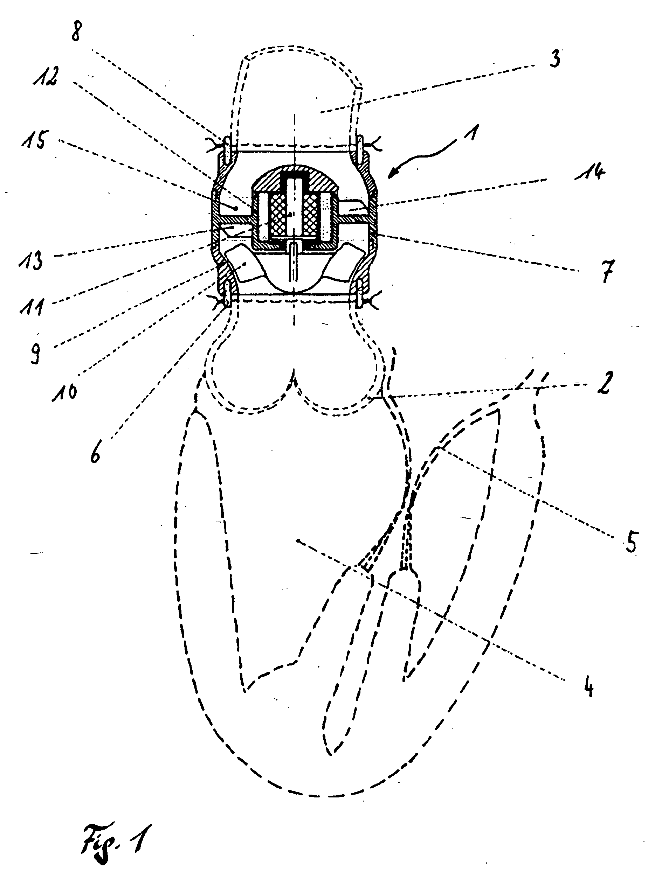

Die in Figur 1 gezeigte Blutpumpe 1 ist zwischen der Aortenklappe 2 und der Aorta ascendens 3 implantiert. Hierbei sind die Bereiche des Herzens und die Aorta ascendens 3 mit gestrichelten Linien dargestellt. Vom Herzen ist die linke Herzkammer 4, die Mitralklappe 5 und die Aortenklappe 2 abgebildet.

Mit einem Nahtring 6 ist das als Pumpengehäuse 7 dienende Conduit in sicherem Abstand zur Aortenklappe 2 angenäht. Das gegenüberliegende Ende des Gehäuses 7 ist mittels eines Nahtringes 8 mit der Aorta ascendens 3 verbunden. Für die Implantation der Blutpumpe 1 wurde ein der Größe des Pumpenconduits entsprechendes Segment der Aorta ascendens reseziert. Die Nahtringe 6 und 8 dienen somit als Gefäßverbindungseinrichtung bei der Implantation der Blutpumpe 1. Die Blutpumpe 1 besteht im wesentlichen aus einem Pumpengehäuse 7, einem darin angeordneten Impeller 10 mit halbaxialer bzw. diagonaler Strömungsführung und einem vollgekapselten Motorantrieb 11. Das Motorgehäuse 12 ist mit dem äußeren Mantel 9 des Pumpengehäuses 7 über Stege 13, 14 fest verbunden. -Diese Stege 13, 14 sind als Strömungsleitschaufeln ausgebildet, in denen die Stromzuführung zum Motorantrieb 11 angeordnet ist. Hierfür sind Kabel (nicht gezeigt) oder Metallstifte (nicht gezeigt) vorgesehen, die innerhalb der als Strömungsleitschaufeln geformten Stege 13, 14 verlaufen.

Grundsätzlich kann jede aus der Strömungsmechanik bekannte Form einer Impellerpumpe mit radialer, halbaxialer oder axialer Strömungsführung und einer entsprechend angepassten Formgebung des Impellers in der Blutpumpe 1 verwendet werden. Ebenso können alle für Blutpumpen beschriebenen Arten der Wellenlagerung, der Wellenabdichtung, der Motorgehäuseabdichtung und der Kraftübertragung, insbesondere auch magnetische Kraftübertragung zum berührungsfreien Antrieb des Impellers verwendet werden. Besonders vorteilhaft ist es, wenn der Antrieb der Blutpumpe 1 durch ein intelligentes System gesteuert wird. Dieses sollte eine Steuerung oder Regelung des arteriellen Blutdruckes und des Druckes im Ventrikel aufweisen.

Des weiteren ist es besonders vorteilhaft, wenn die Steuerung auch einen pulsatilen Betrieb der Impellerpumpe ermöglicht, wobei die Pulsation der Impellerpumpe 1 vorzugsweise mittels Druck- oder EKG-Triggerung an den natürlichen Schlagrhythmus des Herzens adaptiert wird.

Im dargestellten Ausführungsbeispiel ist die Form des äußeren Mantels 9 des Pumpengehäuses 7 so angelegt, dass durch das zentral gelegene Motorgehäuse 12 keine wesentliche Verminderung des freien Strömungsquerschnittes 15 im Sinne einer hämodynamisch relevanten Stenose resultiert.

Im Falle eines Defektes oder im Rahmen einer programmierten Entwöhnungsphase bei Erholung des Herzens kann die Impellerpumpe 1 ganz oder teilweise abgeschaltet werden. Dabei entstehen außer einer fehlenden oder verminderten Herzunterstützung keine wesentlichen Nachteile für die Kreislauffunktion. Im Falle einer gegebenenfalls angezeigten Explantation der Pumpe 1 wird diese durch ein herkömmliches Gefäßinterponat unter Einsatz der Herz-Lungen-Maschine ersetzt.

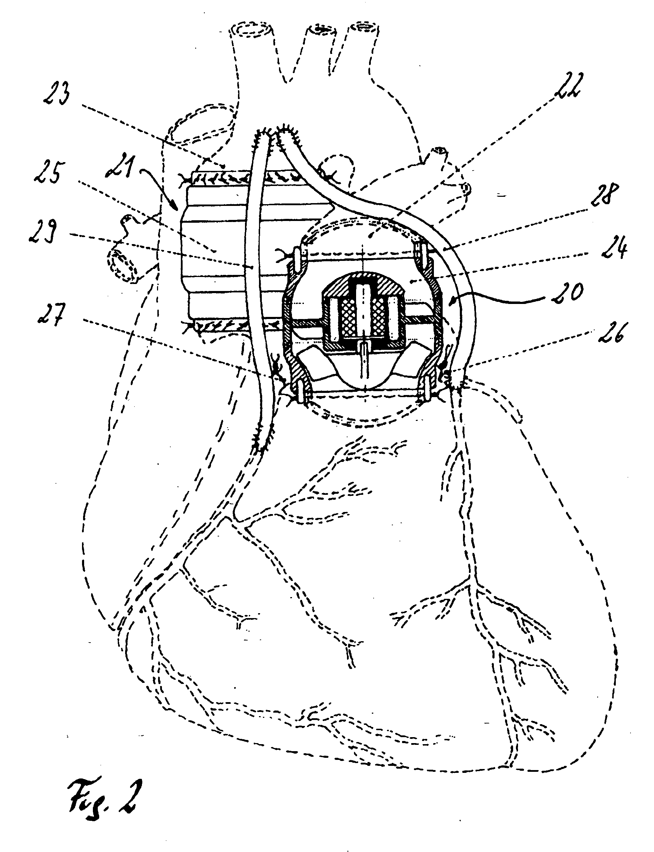

Die Figur 2 zeigt den Einsatz einer Blutpumpe mit zwei Gehäusen 20, 21 zur Entlastung beider Herzkammern. Jeweils in den Truncus pulmonalis 22 und die Aorta ascendens 23 ist ein Pumpenconduit 24, 25 in ausreichendem Abstand zu den Herzklappen implantiert. Hierdurch ist es möglich, eine Teilentlastung aber auch eine vollständige Entlastung des Herzens zu erzielen. Um eine ausreichende Durchblutung der Herzkranzgefäße sicher zu stellen, ist es vorteilhaft, die auf der Niederdruckseite des Pumpenconduits 25 im Bulbus der Aorta abgehenden Stämme der Herzkranzgefäße an den Punkten 26 bzw. 27 zentral zu verschließen und zur Durchblutung der Herzkranzgefäße Venenbypässe 28 und 29 oder Anastomosen mit einer oder beiden Mammaria-Arterien anzulegen.

Die Pumpenconduits 24 und 25 werden vorzugsweise getriggert pulsatil betrieben. Um äußere Momente zu vermeiden, sind die Pumpenconduits 24 und 25 durch ein anpassbares Gestänge (nicht gezeigt) fest miteinander verbunden. Die Impeller werden mit gegenläufiger Rotation betrieben und die Massenträgheitsmomente aller rotierenden Teile sind so ausgelegt, dass durch die Dralländerung bei der Pumpendynamik keine oder nur sehr geringfügige äußere Momente resultieren.

Dieses Prinzip des aktiven Massenausgleichs ist auch bei Pumpen wie in Figur 1 dargestellt mit lediglich einem Pumpengehäuse 7 anwendbar. Hierzu wird an das Pumpengehäuse 7 eine mittels Elektromotor (nicht gezeigt) angetriebene rotierende Masse (nicht gezeigt) konnektiert, welche synchron zum Antriebsmotor 11 der Blutpumpe 1 gegenläufig rotiert und in ihrem Massenträgheitsmoment derart ausgelegt ist, dass durch die Dralländerung bei der Pumpdynamik keine oder nur sehr geringfügige äußere Momente resultieren.

Des weiteren ist es sowohl für die in Figur 1 als auch für die in Figur 2 gezeigte Pumpe vorteilhaft, wenn die Blutpumpen 1 bzw. 20 und 21 durch eine vorzugsweise adaptierbare Aufhängeeinrichtung (nicht gezeigt) an umliegenden Gewebestrukturen, wie etwa dem knöchernen Brustkorb fixiert wird.

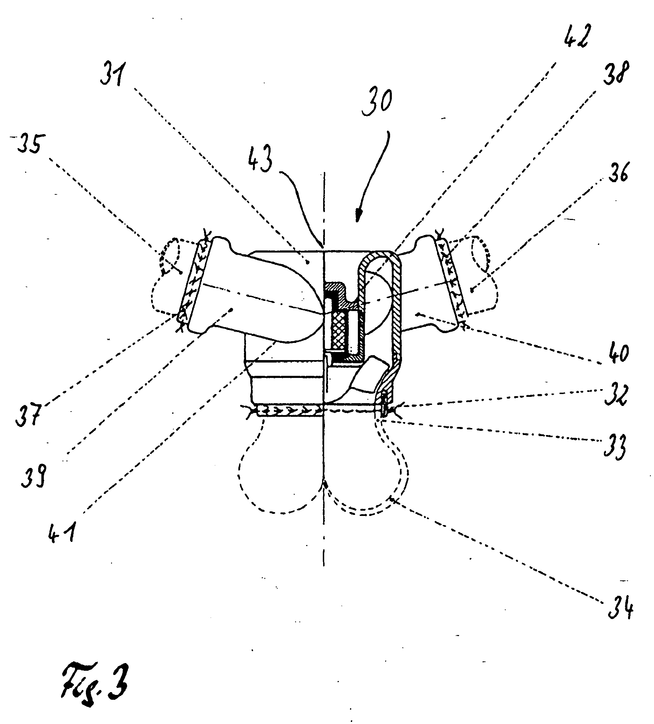

Die in Figur 3 gezeigte Blutpumpe 30 zeigt eine Gestaltungsvariante eines Pumpenconduits 31, das besonders bei sehr kurzem Truncus pulmonalis zur Unterstützung der rechten Herzkammer geeignet ist. Bei dieser Art der Gestaltung ist das Pumpenconduit 31 Y-förmig aufgebaut. Einstromseitig findet sich ein Nahtring 32, welcher analog zu Figur 2 an den Pulmonalarterienstamm 33 unmittelbar hinter der Pulmonalklappe 34 angenäht wird. Abstromseitig werden die rechte und die linke Lungenschlagader 35 bzw. 36 direkt mit Nahtringen 37, 38 an die Auslaufstutzen 39, 40 des Pumpenconduits 31 angeschlossen. Die Auslaufstutzen 39, 40 münden entsprechend der Rotationsrichtung der Pumpe 30 vorzugsweise exzentrisch in das Pumpengehäuse 41. Die Aufhängung des Antriebsmotors 42 im Pumpengehäuse 41 und die Stromzuführung (nicht gezeigt) zum Antriebsmotor 42 können hierbei zentral, beispielsweise an der Stelle 43 erfolgen. Auf die in Figur 1 gezeigten, als Leitschaufeln ausgebildeten Stege 13, 14 kann bei dieser Art der Gestaltung der Blutpumpe 30 verzichtet werden.

Ein gleichartig aufgebautes Pumpenconduit (nicht gezeigt) mit einem den anatomischen Verhältnissen angepassten Winkel zwischen Auslaufstutzen und dem Pumpengehäuse ist auch zur Implantation an der Aortengabel zu beiden Beckenschlagadern zur Unterstützung des Blutflusses bei arterieller Verschlusskrankheit in der Becken- und Beinregion geeignet, wenn rekonstruktive gefäßchirurgische Maßnahmen allein keinen ausreichenden Erfolg bringen.

## Patentansprüche

1. Blutpumpe (1) mit einem Impeller (10) mit mindestens zwei Verbindungseinrichtungen in Form eines Nahtrings oder einer Gefäßprothese (6, 8) zur Interposition in ein Blutgefäß außerhalb des Herzens im Bereich der Aorta ascendens oder des Truncus pulmonalis oder zwischen dem Truncus pulmonalis und den Pulmonalarterien.

2. Blutpumpe nach Anspruch 1, ***dadurch gekennzeichnet, dass*** der Impeller (10) in einem Pumpengehäuse (7) und die Gefäßverbindungseinrichtungen (6, 8) am Pumpengehäuse (7) angeordnet sind.

3. Blutpumpe nach Anspruch 1 oder 2, ***dadurch gekennzeichnet, dass*** die Länge des Pumpengehäuses (7) kürzer als 2 mal vorzugsweise 1,5 mal dessen Durchmesser ist.

4. Blutpumpe nach einem der vorhergehenden Ansprüche, ***dadurch gekennzeichnet, dass*** die Länge der Gefäßverbindungseinrichtungen (6, 8) jeweils kürzer als der Durchmesser des Pumpengehäuses (7) ist.

5. Blutpumpe nach einem der vorhergehenden Ansprüche, ***dadurch gekennzeichnet, dass*** sie einen Motor (11) zum Antrieb des Impellers (10) aufweist und das Pumpengehäuse (7) einen äußeren Mantel (9) aufweist, wobei zwischen dem Motor (11) und dem äußeren Mantel (9) des Pumpengehäuse (7) Stege (13, 14) angeordnet sind, welche vorzugsweise als Leitschaufeln ausgebildet sind.

6. Blutpumpe nach Anspruch 5, ***dadurch gekennzeichnet, dass*** die vorzugsweise als Leitschaufeln ausgelegten Stege (13, 14) den Motor (11) stabil zum äußeren Mantel (9) des Pumpengehäuses (7) halten.

7. Blutpumpe nach Anspruch 5 oder 6, ***dadurch gekennzeichnet, dass*** in den vorzugsweise als Leitschaufeln ausgelegten Stegen (13, 14) Metallkabel oder Metallstifte zur Übertragung von elektrischem Strom angeordnet sind.

8. Blutpumpe nach einem der Ansprüche 5 bis 7, ***dadurch gekennzeichnet, dass*** der freie Strömungsquerschnitt (15) zwischen Motor (11) und dem äußeren Mantel des Pumpengehäuses (7) mindestens 50 %, vorzugsweise mindestens 80 %, des freien Strömungsquerschnitts an einem Ende des Pumpengehäuses (7) beträgt.

9. Blutpumpe nach einem der vorhergehenden Ansprüche, ***dadurch gekennzeichnet, dass*** sie zwei Motoren, zwei Pumpengehäuse (24, 25) und eine vorzugsweise adaptierbare Verbindungseinrichtung zwischen den Pumpengehäusen aufweist.

10. Blutpumpe nach Anspruch 9, ***dadurch gekennzeichnet, dass*** die Impeller gegenläufig antreibbar sind.

11. Blutpumpe nach einem der vorhergehenden Ansprüche, ***dadurch gekennzeichnet, dass*** sie ein Pumpengehäuse (7) mit einem Zusatzmotor aufweist, welcher eine Masse gegenläufig zum Impeller rotierend antreibt.

12. Blutpumpe nach einem der vorhergehenden Ansprüche, ***dadurch gekennzeichnet, dass*** sie ein Pumpengehäuse (7) mit einer Aufhängeeinrichtung zur Befestigung der Pumpe (1, 20, 21, 30) in einem Gewebe, vorzugsweise am knöchernem Brustkorb, aufweist.

## Claims

1. A blood pump (1) with an impeller (10) with at least two connecting devices in the form of a suture ring or a vascular prosthesis (6, 8) for interposition inside a blood vessel outside the heart, in the area of the ascending aorta or the pulmonary trunk or between the pulmonary trunk and the pulmonary arteries.

2. The blood pump according to claim 1, ***characterized in that*** the impeller (10) is disposed in a pump casing (7) and the vascular connecting devices (6, 8) are disposed on the pump casing (7).

3. The blood pump according to claim 1 or 2, ***characterized in that*** the length of the pump casing (7) is shorter than 2 times, preferably 1,5 times its diameter.

4. The blood pump according to one of the afore-mentioned claims, ***characterized in that*** the length of the vascular connecting devices (6, 8) is respectively shorter than the diameter of the pump casing (7).

5. The blood pump according to one of the afore-mentioned claims, ***characterized in that*** it has a motor (11) for driving the impeller (10) and that the pump casing (7) has an outer shell (9), webs (13, 14) that are preferably configured as guide vanes, being disposed between the motor (11) and the outer shell (9) of the pump casing (7).

6. The blood pump according to claim 5, ***characterized in that*** the webs (13, 14) preferably designed as guide vanes hold the motor (11) steady to the outer shell (9) of the pump casing (7).

7. The blood pump according to claim 5 or 6, ***characterized in that*** metal cables or metal pins for transmitting electric current are disposed in the webs (13, 14) preferably designed as guide vanes.

8. The blood pump according to one of the claims 5 to 7, ***characterized in that*** the clear flow cross-section (15) between the motor (11) and the outer shell of the pump casing (15) amounts to at least 50%, preferably 80% of the free flow cross-section at one end of the pump casing (7).

9. The blood pump according to one of the afore-mentioned claims, ***characterized in that*** it has two motors, two pump casings (24, 25) and a preferably adaptable connecting device between the pump casings.

10. The blood pump according to claim 9, ***characterized in that*** the impellers are adapted to be driven in counterrotation.

11. The blood pump according to one of the afore-mentioned claims, ***characterized in that*** it has a pump casing (7) with an additional motor that rotationally drives a mass in the opposite direction of the impeller.

12. The blood pump according to one of the afore-mentioned claims, ***characterized in that*** it has a pump casing (7) with a hanging device for fixing the pump (1, 20, 21, 30) to a tissue, preferably to the bones of the thorax.

## Revendications

1. Pompe à sang (1) avec une roue à aubes (10) avec au moins deux systèmes de liaison sous forme d'un anneau de suture ou d'une prothèse vasculaire (6, 8), destinée à être interposée dans un vaisseau sanguin à l'extérieur du coeur dans la zone de l'aorte ascendante ou du tronc pulmonaire ou entre le tronc pulmonaire et les artères pulmonaires.

2. Pompe à sang selon la revendication 1, ***caractérisée en ce que*** la roue à aubes (10) est disposée dans un boîtier de pompe (7) et que les systèmes de liaison vasculaires (6, 8) sont disposés sur le boîtier de pompe (7).

3. Pompe à sang selon la revendication 1 ou 2, ***caractérisée en ce que*** la longueur du boîtier de pompe (7) est plus courte que 2 fois, de préférence, 1,5 fois son diamètre.

4. Pompe à sang selon l'une quelconque des revendications précédentes, ***caractérisée en ce que*** la longueur des systèmes de liaison vasculaires (6, 8) est respectivement plus courte que le diamètre du boîtier de pompe (7).

5. Pompe à sang selon l'une quelconque des revendications précédentes, ***caractérisée en ce qu*'**elle comporte un moteur (11) pour entraîner la roue à aubes (10) et que le boîtier de pompe (7) comporte une gaine externe (9), des traverses (13, 14), configurées de préférences comme aubes directrices, étant disposées entre le moteur (11) et la gaine externe (9) du boîtier de pompe (7).

6. Pompe à sang selon la revendication 5, ***caractérisée en ce que*** les traverses (13, 14) conçues comme aubes directrices maintiennent le moteur (11) par rapport à la gaine externe (9) du boîtier de pompe (7).

7. Pompe à sang selon la revendication 5 ou 6, ***caractérisée en ce que*** des câbles ou des tenons métalliques pour la transmission de courant électrique sont disposés dans les traverses (13, 14) configurées comme aubes directrices.

8. Pompe à sang selon l'une quelconque des revendications 5 à 7, ***caractérisée en ce que*** la section de passage libre (15) entre le moteur (11) et la gaine externe du boîtier de pompe (7) vaut au moins 50%, de préférence au moins 80% de la section de passage libre à l'extrémité du boîtier de pompe (7).

9. Pompe à sang selon l'une quelconque des revendications précédentes, ***caractérisée en ce qu'***elle comporte deux moteurs, deux boîtiers de pompe (24, 25) et un système de liaison de préférence adaptable entre les boîtiers de pompe.

10. Pompe à sang selon la revendication 9, ***caractérisée en ce que*** les roues à aubes sont susceptibles d'être entraînées en rotation inverse.

11. Pompe à sang selon l'une quelconque des revendications précédentes, ***caractérisée en ce qu*'**elle comporte un boîtier de pompe (7) avec un moteur supplémentaire, lequel entraîne une masse en sens inverse par rapport à la roue à aubes.

12. Pompe à sang selon l'une quelconque des revendications précédentes, ***caractérisée en ce qu'***elle comporte un boîtier de pompe avec un système de suspension pour fixer la pompe (1, 20, 21, 30) dans un tissu, de préférence sur le thorax osseux.
